# EUROPEAN PATENT APPLICATION

(11) **EP 0 549 267 A1**
(43) Date of publication of application: **30.06.1993**
(21) Application number: 92311553.9
(22) Date of filing: 17.12.1992
(51) Int. Cl.: A61K 7/025, A61K 7/027

(54) **Lip treatment composition**

(30) Priority: 20.12.1991 GB 9127062
(71) Applicant: UNILEVER PLC, London EC4P 4BQ (GB); UNILEVER N.V., NL-3013 AL Rotterdam (NL)
(72) Inventor: Dunphy, Patrick Joseph, Sharnbrook, Bedford MK44 1LQ (GB); Dunnett, Paul, Sharnbrook, Bedford MK44 1LQ (GB); Lillford, Peter John, Sharnbrook, Bedford MK44 1LQ (GB)
(74) Representative: Mulder, Cornelis Willem Reinier, Dr.

(57) **Abstract**

A lip-treatment composition for topical application to the lips comprising a base comprising oil, water and a structurant, and at least one active component active with regard to the lips, gums, teeth, throat, or oral mucosa.

## Description

This invention relates to a cosmetic emulsion treatment composition adapted to be applied to the lips, for depositing thereon a therapeutic and/or protective film, which may or may not be coloured. The composition is especially be used for the delivery of active components to the mouth or throat, in particular to the teeth and gums.

### Background and Prior Art

Lip treatment compositions of various types are known from the art. These may be pigmented, such as are commonly found in lipstick compositions, or unpigmented, such as are typically found as lip balm or lip gloss treatments.

Usually these compositions are anhydrous, although water containing lip treatment compositions are known. For example, JP-A-61/83110 (Konuki) describes a lip treatment composition, primarily for use as a lipstick, which is a fat/oil coating containing water.

Moreover, US 4,770,871 (Greenshields) describes the incorporation of mono- and dialkyl-1,4:3,6-dianhydrosorbitol blends in lip creams, these compounds being useful as plaque inhibiting dentifrice compositions. However, this reference contains no examples which show a composition of any such lip creams, and no mention of such lip treatment composition containing water.

### Summary of the invention

We have discovered that by employing active components, in particular water soluble active components, in a water-containing lip treatment it is possible to deliver these active components to the mouth and throat, primarily by the action of the wearer licking his or her lips. These active components may especially be dental active components.

Moreover, because the lip treatment composition contains water, it is possible for the active components to be delivered to the mouth in solubilised form, in which form they often have a greater activity and availability to the mouth and throat, rather than in a suspended form in which they may be less effective.

### Definition of the Invention

Thus according to the invention there is provided a topical composition suitable for application to the lips, comprising a base comprising oil, water and a structurant, and at least one active component active with regard to the lips, gums, teeth, throat, and oral mucosa.

In a preferred embodiment, the at least one active component is one active with regard to the teeth or gums

A preferred product form of compositions according to the invention is a stick, though the compositions may be used in the form of a cream. Accordingly the composition preferably comprises sufficient structurant to provide a product in stick form. However, the amount of structurant required for this may vary according to the type of structurant used.

### Disclosure of the invention

The invention is concerned with the provision of active compounds to the lips, mouth, throat, teeth and oral mucosa, in particular the provision of dental active compounds to the teeth and mouth. By providing the active compounds in a water-containing lip treatment composition, the active compounds are effectively dosed to the mouth at a low but regular rate through the action of the wearer of the composition licking his or her lips, and also eating or drinking. By "active" in this context is meant a compound which provides a benefit to target area, which may include the treatment of symptoms and the provision of compounds which provide a longer term health benefit.

The composition is particularly useful for the administration of dental active compounds such as soluble or sparingly soluble fluorides, an optimal administration of which is to expose the teeth to a constant but low level. Thus the provision of a fluoride compound which in water is soluble or sparingly soluble in a water-containing lip treatment (which allows the fluoride to be administered in a solubilised form) is a particularly advantageous form of administration.

The lip treatment composition according to the invention can also be used to deliver to the lips skincare active ingredients, as hereinafter disclosed, which can function to provide moisturisation, emolliency, protection from the elements, and other benefits conventionally associated with lip treatment compositions, such as reparative and restorative properties.

Preferably, the composition is structurally a water-in-oil composition, but alternatively it is possible to provide a product form in accordance with the invention which is an oil-in-water emulsion.

### The structurant

The structurant may comprise any conventionally used structurant, including gelling or thickening agents such as proteins, polysaccharides and starch as well as synthetic polymers, and emulsifiers, which may contribute to the stability and pleasing appearance of the composition, and also help provide the physical structure and water retention properties required of the lip treatment composition. The amount of structurant required in the composition may vary according to the type of structurant or structurants employed, but may typically total 0.1-35% of the composition.

Examples of gelling agents include :
- heat setting biopolymers, for example albumin;
- cool setting biopolymers, such as gelatin or agar;
- thickening biopolymers, for example xanthan, starch, guar, or locust bean gum;
- known synergistic mixtures of biopolymers, such as locust bean gum of guar gum with xanthan;
- thickening/gelling synthetic polymers, for example carbopols;
- chemically setting biopolymers of defined melting point. In this context, it is known that the melting point of various chemically setting biopolymers can be adjusted to a desired level by incorporating into these biopolymers the appropriate amount of chemical reagent, for example (in the case of carrageenan gum) the appropriate level of potassium or sodium ion. By varying the melting point of the chemically setting biopolymer thus employed, the feel of the cosmetic composition may be altered, in particular the feel on application.

Examples of chemically setting biopolymers, and the chemical reagent, the concentration of which determines the melting point of the chemically setting biopolymer include :

| Chemically setting biopolymer | Chemical reagent |
|---|---|
| kappa-carrageenan | potassium and/or sodium ion |
| iota-carrageenan | potassium and/or sodium ion |
| alginate | calcium and/or sodium ion |
| pectin | sugar |
| low-methoxy pectin | calcium ion |
| chemically modified starch | eg acetic, adipic or citric cross-linked |

One or more of these thickening or gelling agents may be employed as the structurant. The total amount of thickening or gelling agent in the system may comprise 0.1-10%, more preferably 0.2-3%, of the total cosmetic composition.

The structurant may additionally or alternatively comprise one or more emulsifiers. Preferred emulsifiers include phospholipids or phospholipid derivatives, glyceride or glyceride derivatives, or mixtures thereof.

Classes of preferred phospholipids include phosphoglycerides, lysophosphoglycerides, and sphingomyelins.

Preferred phospholipids include phosphatidyl choline, phosphatidyl ethanolamine, phosphatidyl serine, phosphatidyl inositol, disphosphatidy glycerol and ammonium phosphatides. However, the most preferred phosphoglyceride is lecithin, in particular soybean lecithin, which comprises a mixture of some of the above examples of specific phosphoglycerides.

When phospholipids are used in compositions according to the invention, they are preferably used at a level of 0.2 to 30%, more preferably 0.2 to 15%, most preferably 0.2 to 2% by weight of the composition.

Preferred lysophosphoglycerides include lysophosphatidyl choline, lysophosphatidyl ethanolamine. lysophosphatidyl serine, and lysophosphatidyl inositol, or mixtures thereof.

Another category of preferred emulsifiers include acyl glycerol derivatives. Preferred derivatives include glyceryl monoalkanoates and glyceryl monoalkenoates, the organic side-chain group having 2 to 20 carbon atoms, with or without one or more hydroxyl groups, and being branched or unbranched. Examples include glyceryl caprylate, glyceryl caproate, glyceryl laurate, glyceryl palmitate, glyceryl stearate, glyceryl oleate, glyceryl linoleate and glyceryl ricinoleate.

Another preferred category of acyl glycerol derivatives is di (e.g. 1,2- and/or 1,3-) substituted glyceryl derivatives, such as glyceryl dialkanoates, glyceryl dialkenoates, or mixed esters. Preferably, the substituted moieties have 3 to 20 carbon atoms and may be branched or unbranched, with or without one or more hydroxyl groups. Specific examples of such di-substituted glycerols include glyceryl diacetate, glyceryl dibutanoate, glyceryl dilaurate, glyceryl dioleate, glyceryl dilinoleate, and glyceryl oleyl, palmitate.

A further preferred category of glycerol derivatives are polyglyceryl esters having acyl groups of 2 to 20 carbon atoms, either branched or unbranched, and with or without one or more hydroxyl groups, such as, for example, polyglyceryl ricinoleate, stearate and distearate.

Other preferred glycerol derivatives include glyceryl ether derivatives, polyhydric ethers, and polyhydric alcohols.

Particularly preferred examples of the glyceride derivative emulsifiers are glyceryl caprate, glyceryl laurate, glyceryl myristate, glyceryl oleate, glyceryl linoleate, glyceryl isostearate, glyceryl dilinoleate and glyceryl dicaprate. When glyceride derivatives are used in compositions according to the invention, they are preferably used at a level of from 0.2 to 35%, more preferably 0.5 to 10%, most preferably 0.5 to 5% by weight of the composition.

When the composition comprises a mixture of phospholipids or phospholipid derivatives, and glycerides or glyceride derivatives, preferably these combined are present in the composition at a level of 0.2-20% by weight of the composition, more preferably 0.2-10% by weight of the composition.

Where an emulsifier is employed in the composition, it is employed at a level of from 0.2 to 35%, more preferably from 0.2 to 10%, more preferably around 3-4 % by weight of the composition.

### The Active Component

Any type of component or combination of actives may be incorporated into the lip-treatment composition for which slow release into the oral cavity is desirable. By "active component" is meant a substance that is present in the composition as applied to the lips, and which may act as a direct treatment for the lips (for example for cold sores or chapped lips). Conversely it may be applied to the lips, and is then delivered by the licking, eating or drinking action of the user to another part of the mouth or throat where it delivers a benefit. These include both lipid and water soluble active components, for the treatment of the lips, gums, teeth, oral mucosa (for example for the purpose of treating breath malodour) and the throat.

However, because the lip composition contains water, it is particularly useful for delivering active components that are water-soluble or at least sparingly soluble in water, thus potentially increasing the effectiveness of the water-soluble active so applied. The chemical system employed in compositions according to the invention may be both water and lipid based.

Preferred active compounds for incorporation into the composition are dental active compounds, which may be especially active towards the teeth and gums, and which include :
- anti-caries compounds, including soluble and sparingly soluble compounds, such as fluorides and monofluorophosphates. Specific examples include sodium fluoride, calcium fluoride, zinc fluoride, stannous fluoride, and sodium monofluorophosphate;
- anti-calculus agents, such as pyrophosphates;
- anti-plaque agents, such as trimetaphosphate;
- metal ions, in particular copper, tin and zinc and their salts (for example citrates, acetates, oxides and stearates);
- cationic agents, such as cetyl pyridinium chloride;
- chlorhexidine gluconate;
- anti-gingivitis agents, such as potassium nitrate
- anti-inflammatory agents, such as the corticosteroids, phenacetin, and aspirin; or mixtures thereof.

Lipid soluble actives that may advantageously be included in compositions according to the invention include triclosan, salicylanilide and dichlorobenzyl alcohol.

Other active components that may advantageously be incorporated in compositions according to the invention include biological active compounds. These include for example antibodies and/or antibody fragments, enzymes, and antibiotics, such as lysozyme, glucose oxidase, lactoperoxidase and nisin.

Preferred active components for incorporation into compositions according to the invention are components which are soluble or sparingly soluble in water. By "sparingly soluble", we mean that it has a solubility in water at 25°C of greater than about 0.005% by weight. Especially preferred active components for incorporation into compounds according to the invention are anticaries agents containing a fluoride which is soluble or sparingly soluble in water, and metal ions such as zinc and its salts.

The active component may be present in the composition at a sufficient level so as to deliver a benefit to the target level. Such a level may typically be 0.005-5% by weight of the composition, more preferably 0.1-1% by weight of the composition.

### The Oil

The oil used in the composition may be only oil which is used in cosmetic compositions. Suitable oils include but are not limited to;
caprylic triglycerides
capric triglycerides
isostearic triglycerides
adipic triglycerides
propylene glycol myristyl ether acetate
lanolin oil
polybutene
isopropyl palmitate
isopropyl myristate
diethyl sebacate
diisopropyl adipate
hexadecyl stearate
cetyl oleate
oleyl alcohol
hexadecyl alcohol
wheatgerm oil
hrydrogenated vegetable oils
petrolatum
modified lanolins
branched-chain hydrocarbons, alcohols and esters
castor oil
corn oil
cotton seed oil
olive oil
palm kernel oil
rapeseed oil
safflower seed oil
jojoba oil
evening primrose oil
avacado oil
mineral oil
volatile and non-volatile silicone oils.

The amount of the oil normally present in the cosmetic emulsion of the invention is from 2 to 97%, preferably 30 to 90% by weight of the cosmetic emulsion.

The cosmetic emulsion of the invention can optionally also comprise one or more waxy ingredients, hereinafter referred to as a "wax".

Examples of waxes include:
candelilla wax
ozokerite wax
carnauba wax
beeswax
spermaceti
lanolin
cetyl alcohol
stearyl alcohol
high melting point parafinic hydrocarbons.

The amount of wax present in the composition of the invention when used is up to about 25%, usually from 1 to 25%, and preferably from 5 to 20% by weight of the composition.

The cosmetic emulsion of the invention can optionally also comprise other ingredients as conventionally employed in lipsticks or other lipcare products.

Examples of other ingredients include perfumes, antioxidants, colorants, such as staining dyes and pigments, humectant, germicides, sunscreens, lipid materials, and vitamins.

Particularly preferred pigments, when present, include calcium, barium and aluminium lakes, ion oxides, titanium dioxide, and mica.

Particularly preferred humectant include glycerol, sorbitol and other polyols.

Particularly preferred germicides include Triclosan.

Particularly preferred sunscreens include octyl methoxycinnamate and butyl methoxydibenzoylmethane.

Particularly preferred lipid materials include ceramides and liposomes.

Particularly preferred vitamins include vitamin A, C, D and E.

### Examples

The invention will now be further demonstrated by way of example only.

### Example 1

| Component | %w/w |
|---|---|
| Candelilla wax | 7.9 |
| Ozokerite wax | 3.0 |
| Beeswax | 4.9 |
| Carnauba wax | 0.9 |
| Lanolin | 5.0 |
| Caprylic/Capric triglyceride | 5.8 |
| Propylene glycol myristyl ether acetate | 6.0 |
| Caprylic/Capric/Adipic/Isostearic triglyceride | 9.0 |
| Isopropyl palmitate | 11.0 |
| Antioxidant | 0.1 |
| Monoglyceride | 3.5 |
| Soya lecithin | 1.0 |
| Polybutene | 0.8 |
| Lanolin oil | 2.3 |
| Castor oil | 26.2 |
| Triclosan | 0.1 |
| Fragrance | 0.1 |
| Titanium dioxide | 0.3 |
| Sodium fluoride | 0.5 |
| Zinc citrate | 0.3 |
| Water | 11.3 |

### Preparation

The lipid phase components were mixed, and melted at 85°C. To this was added with stirring titanium dioxide dispensed in castor oil. The mixture is then cooled to 75°C, and to this was added with vigorous homogenisation a hot (75°C) aqueous solution of the hydrophillic ingredients. The solution was then degassed whilst still warm, and moulded with cooling.

The resultant treatment stick was found to have pleasing properties on application, but can also provide for a slow, low level of delivery of fluoride to the mouth.

### Example 2

The following provides an example of a biologically active treatment composition for application to the lips.

The composition is prepared the same as that in example 1, except that it does not contain the fluoride, zinc citrate or triclosan. Instead, the composition contains a biological system based on the enzyme lysozyme (containing approximately 500 ppm/ml in an aqueous phase), in combination with the polypeptide antibiotic nisin (500 units/ml in an aqueous phase). The preparation of the composition is by the same method as described in example 1, except the biological system, which is dispersed in the aqueous phase, is homogenised with the other hydrophillic components at 55°C or less. Alternatively, the biological system can be incorporated into a generally softer hydrophillic base, to maintain the stability of the enzyme.

The resulting composition, when applied to the lips, provides for satisfactory delivery of the biological system to the mouth.

### Example 3

The base composition and preparation method as described in example 2 is used. The active antibacterial ingredients incorporated into the aqueous phase can however be glucose oxidase and lactoperoxidase, at levels of 2-5 and 10 units/ml of an aqueous phase respectively. The antibacterial system exhibits generally low toxicity towards eucaryotic cells and yeast, and has significant potential for oral systems.

### Experimental

A composition based on example 1 according to the invention may be assessed for its delivery of active components such as zinc and fluoride to the oral cavity according to the following protocol.

Using a panel of test personnel (typically 7), background levels of zinc in a panellists saliva are established by collecting from each panellist a sample of 5ml saliva in a suitable container, and analysing this for its zinc content by conventional analytical techniques using atomic absorption spectroscopy and electrochemical analysis.

On an assessment day panellists are asked not to clean their teeth in the morning. At the start of the assessment a stick composition according to the invention and containing 0.5% zinc citrate and 0.8% sodium monofluorophosphate is liberally applied to the lips (approx 20-30mg). Saliva samples are then collected from the panellists, who are instructed to behave and lick their lips as they would do normally, at intervals of 1 minute, 5 minutes, 15 minutes, 30 minutes, 1 hour and 2 hours from the start of the test. Samples are then analysed for zinc and fluoride levels by conventional analytical techniques using atomic absorption spectroscopy and electrochemical analysis. Samples may be stored if necessary at -10°C for up to 24 hours before analysis.

An increased level of active components in the mouth can be observed.

## Claims

1. A lip-treatment composition for topical application to the lips comprising a base comprising oil, water and a structurant, and at least one active component active with regard to the lips, gums, teeth, throat, or oral mucosa.

2. A lip-treatment composition according to claim 1, wherein the composition is in stick form.

3. A lip treatment composition according to claim 1 or claim 2, wherein the structurant comprises a thickening or gelling agent.

4. A lip treatment composition according to any of the preceding claims, wherein the composition comprises an emulsifier.

5. A composition according to claim 4 wherein the composition comprises 0.2-35% by weight of an emulsifier.

6. A composition according to claim 4 or claim 5, wherein the emulsifier comprises a phospholipid or a phospholipid derivative, and a glyceride or a glyceride derivative.

7. A composition according to claim 6 wherein the emulsifier is present in the composition at a level of from 0.2-20% by weight of the composition.

8. A composition according to any of the preceding claims, wherein the at least one active component comprises an anticaries compound, an anti-calculus agent, an anti-plaque agent, a metal ion, a cationic agent, chlorhexidine gluconate, an anti-gingivitis agent or an anti-inflammatory agent, or mixtures thereof.

9. A composition according to claim 8 wherein the active component is a metal ion or an anticaries compound.

10. A composition according to any of the preceding claims, wherein the active component is soluble or sparingly soluble in water.

11. A composition according to any of the preceding claims wherein the active component is zinc or fluoride.
